(19) Europäisches Patentamt / European Patent Office / Office européen des brevets

(11) **EP 1 993 505 B1**

(12) **EUROPÄISCHE PATENTSCHRIFT**

(45) Veröffentlichungstag und Bekanntmachung des Hinweises auf die Patenterteilung:
**26.08.2009 Patentblatt 2009/35**

(21) Anmeldenummer: 07712370.1

(22) Anmeldetag: **28.02.2007**

(51) Int Cl.:
*A61K 8/89* (2006.01)      *A61Q 5/06* (2006.01)

(86) Internationale Anmeldenummer:
**PCT/EP2007/051882**

(87) Internationale Veröffentlichungsnummer:
**WO 2007/104645 (20.09.2007 Gazette 2007/38)**

(54) **HAARPFLEGEZUSAMMENSETZUNG**

HAIRCARE COMPOSITION

COMPOSITION DE SOINS CAPILLAIRES

(84) Benannte Vertragsstaaten:
**BE DE FR GB NL**

(30) Priorität: **16.03.2006 DE 102006012199**

(43) Veröffentlichungstag der Anmeldung:
**26.11.2008 Patentblatt 2008/48**

(73) Patentinhaber: **Wacker Chemie AG**
**81737 München (DE)**

(72) Erfinder:
• **SCHWARZWÄLDER, Claudius**
**84489 Burghausen (DE)**
• **ZARKA, Michael Tobias**
**84489 Burghausen (DE)**
• **HÖGL, Christian**
**84367 Reut (DE)**
• **SCHWARZWÄLDER, Heidemarie**
**84489 Burghausen (DE)**

(74) Vertreter: **Fritz, Helmut et al**
**Wacker Chemie AG**
**Zentralbereich Patente, Marken und Lizenzen**
**Hanns-Seidel-Platz 4**
**81737 München (DE)**

(56) Entgegenhaltungen:
EP-A2- 0 412 704      WO-A-03/085035
WO-A-20/04065437

EP 1 993 505 B1

**Beschreibung**

Diese Erfindung betrifft Haarpflegezusammensetzungen, mit verbesserten Auftrageigenschaften und verbesserten Eigenschaften in Kombination aus Halt, Flexibilität und Weichgriff.

Die Schriften The History of Polymers in Hair Care (1940-Present) und R.Y. Lochhead, Cosmetic&Toiletries, p.23, 103 (1988) zeigen, dass die Entwicklung von Frisuren und Haar Styling Produkten schon seit langem durch die Weiterentwicklung von Polymeren beeinflusst und durch deren neuen Eigenschaften geprägt wird.

Von Schellack über PVP wurden vor allem durch den Einsatz von Co-Polymeren die Eigenschaften gezielt, durch die einzelnen Monomerbausteine, auf die gewünschten Haar Styling Effekte eingestellt und kontinuierlich verbessert. Ein klassisches Beispiel sind die PVA/PVP Co-polymere, bei denen die Hygroskopie des PVP durch Einbau von Vinylacetaten auf ein vorteilhaftes Maß eingestellt werden konnte.

Dieser stetigen Weiterentwicklung folgten auch immer weitere Anforderungen, denen neuen Produkte genügen müssen. Beispiele für solche Anforderungen an ein Haarpflegemittel sind Verbesserter Halt der Frisur (bessere Fixierung), kein klebriges Gefühl, kein steifes Haar, schnelle Trocknungszeiten, leichte Kämmbarkeit, Haarvolumen, kein Beschweren der Haare, keine Wasserabsorption durch das.Polymer, leichte Anwendbarkeit im nassen Haar, leichte Auswaschbarkeit mit einem Shampoo und keine Schuppenbildung des Polymerfilms beim Auskämmen.

Wegen der Vielzahl an Anforderungen an so ein Produkt, mit teilweise gegensätzlichen Eigenschaften, wundert es daher nicht, dass es eine Vielzahl von Polymeren für Haar Styling Anwendungen gibt.

Als ein dominantes Problem stellt sich die Kombination von guter Fixierung der Frisur und gleichzeitigem nichtklebrigen - oder besser- weichem/natürlichem Griff des Haares dar. Insbesondere wenn Polymere für starken Halt wie Acrylate, Acetate, Methylvinyl Ether/ Maleinsäure-anhydrid oder PVP eingesetzt werden.

Um einen flexiblen Halt zu bekommen, der der Frisur noch natürliche Bewegung ermöglicht und kein steifes Haar mit einem spröden Polymerfilm erzeugt, werden Copolymere aus einer Kombination von Acrylat- und Silikonteil eingesetzt, wobei der hochflexible Silikonteil neben hoher Hydrophobie auch einen weichen Griff einbringt.

Es gab verschiedene Lösungsansätze um die Verknüpfung dieser Bausteine zu erreichen und sie wurden für Haar Styling Anwendungen insbesondere Haar Spray Anwendungen beschrieben.

So werden in der Anmeldung EP 0 408 311 A2 Silikoncopolymere mit mindestens 15 Gew.-% hydrophilem Monomer beansprucht, wie beispielsweise einem Dimethylaminoethylmethacrylat, um eine Fixierung zu bekommen. Das US-Patent US 5,565,193 beschreibt eine Formulierung die zusätzlich noch 0,5 bis 15 Gew.-% eines Kohlenwasserstoff-Lösungsmittels enthält mit einem Siedepunkt >105°C. In den Schriften WO 95/00106 und WO 99/55294 werden Formulierungen mit einem Wassergehalt von höchstens 10% beschrieben. In weiteren Patentschriften US 6,350,439 B1, US 2002/0015681 A1, US 5,618,524 und EP 0 412 704 B1 sowie WO 00/51557 werden zudem Propf-Copolymere mit monofunktionalen Silikonmacromonomeren für den Einsatz in Haar Styling Produkten beschrieben, die die oben ausgeführte Effektkombination erzielen.

Obwohl diese Haarspray Formulierungen mit Silikoncopolymeren bereits zu einer gewissen Verbesserung der Kombination gute Fixierung und Styling mit angenehmerem Haarweichgriff führen, ist eine weitere Verbesserung der Einzeleffekte in Kombination erforderlich, da sie vom Konsumenten nachgefragt wird. Da neben den chemischen Blöcken, die das Haarstylingpolymere aufbauen, und so Einfluss auf die Fixierung und den Weichgriff genommen werden kann, zusätzlich auch das rheologische Verhalten der Haarspraylösung, der Tröpfchengröße des Sprays und der Viskosität des Polymerlösungströpfchens auf dem Haar einen großen Einfluss auf die gewünschten Effekte haben, muss hier eine Zusammensetzung gefunden werden, die auch zu einer Verbesserung der Parameter führt und damit zu einer Steigerung der Effekte Fixierung und Halt.

Niederviskose Zusammensetzungen verteilen sich aufgrund ihrer besseren Spreitung leichter auf der Haaroberfläche. Bekannt ist, dass je niedriger die Viskosität der Haarsprayformulierung auf dem Haar ist, desto unauffälliger ist das klebrige Empfinden und umso angenehmer fühlt sich das Haar an.

Eine niedrige Viskosität der Haarspraylösung führt aber beim Sprühvorgang zu kleineren Tröpfchengrößen. Kleine Tröpfchen erreichen aber eine schwächere Fixierung zwischen den einzelnen Haarfasern und somit einen geringen Halt der Frisur, während größere Tröpfchen zu einer stärkeren Fixierung und damit zu einem besseren Halt der Frisur beitragen.

Optimal sind also Haarsprays die beim Versprühen große Tröpfchen aufweisen aber eine auf dem Haar ankommende Polymerlösung mit niedriger Viskosität haben.

Die Tröpfchengrößen kann bis zu einem gewissen Grad durch eine geeignete Wahl an Ventilen und Sprühköpfen eingestellt werden. Letztendlich beeinflussen aber die rheologischen Parameter der Polymerlösung und des Polymerfilms die hauptsächliche Verbesserung der Effekte.

Um eine Verbesserung der Fixierung und Flexibilität unter Berücksichtigung der beschrieben Problematik zu erhalten, beschreiben die Autoren in WO 00/51557 und US 2002/0015681 A1 eine Zusammensetzung, die eine Kombination aus zwei verschiedenen Silikon Propfcopolymeren beinhaltet. Die Zusammensetzung weist dadurch eine hohe Viskosität auf, wenn sie unter hoher Scherung aus dem Spraybehälter entlassen wird und eine niedrige Viskosität, wenn sie auf dem Haar ankommt und einer geringen Scherung ausgesetzt ist. Nachteilig ist hier jedoch, dass für die Formulierung zwei verschiedene Polymere eingesetzt werden müssen. Die Polymere haben ein hohes Molekulargewicht was zu hohen Lösungsviskositäten führt und somit zu einer schwierigern Verarbeitung. Wünschenswert wäre daher die Verwendung eines einzigen Polymers welches zudem einen niedrigeren Molekulargewichtsbereich aufweist.

Aufgabe dieser Erfindung war es daher eine Haarpflegezusammensetzung zur Verfügung zu stellen, die sowohl verbesserte Verarbeitungseigenschaften bei der Herstellung als auch verbesserte Anwendungseigenschaften in Kombination aus Halt, Flexibilität und Weichgriff aufweist.

Gegenstand der Erfindung sind daher Haarpflegezusammensetzungen, **dadurch gekennzeichnet, dass** sie 0,1-12 Gew.-% mindestens eines Silikoncopolymers und/oder dessen Verseifungsprodukte enthalten, welches aus

A) 0,1-50% eines oder mehrerer Silikone, wobei 10-100% dieser Silikone mindestens zwei polymerisierbare Gruppen besitzen,
B) 0,5-14% eines oder mehrerer hydrophiler Monomere und
C) 30-99,4% eines oder mehrerer hydrophober Monomere besteht,

mit der Massgabe, dass das Melekulargewicht $M_W$ des Silikonpolymers 30.000 bis 90.000 g/mol beträgts. Bevorzugt besteht das Silikoncopolymer aus 0,1-40% Silikon A).

Diese Art von Silikoncopolymeren und ein Verfahren zu deren Herstellung wurden bereits in der Patentschrift WO 03/085035 A1 beschrieben. Unerwartet zeigte sich jedoch, dass die in der WO 03/085035 A1 lediglich als optional beschriebenen Hilfsmonomere, für die erfindungsgemäßen Haarpflegezusammensetzung zwingend notwendig sind, da erst 0,5-14%, bevorzugt 0,5-10%, besonders bevorzugt 3-6%, hydrophile Monomere B) den wesentlichen Beitrag leisten, um die Eigenschaften in Kombination aus Halt, Flexibilität und Weichgriff überhaupt erst zu verbessern.

Zur Herstellung der Verseifungsprodukte wird das Silikoncopolymer in dem Fachmann bekannter Weise in alkoholischer Lösung verseift, wobei die dabei üblichen sauren oder alkalischen Katalysatoren eingesetzt werden. Geeignete Lösungsmittel sind aliphatische Alkohole mit 1 bis 6 C-Atomen, vorzugsweise Methanol oder Ethanol. Die Verseifung kann aber auch in einer Mischung bestehend aus Wasser und aliphatischem Alkohol durchgeführt werden. Saure Katalysatoren sind beispielsweise starke Mineralsäuren, wie Salzsäure oder Schwefelsäure, oder starke organische Säuren, wie aliphatische oder aromatische Sulphonsäuren. Bevorzugt werden alkalische Katalysatoren eingesetzt. Dies sind beispielsweise die Hydroxide, Alkoholate und Carbonate von Alkali- oder Erdalkalimetallen. Die Katalysatoren werden in Form von deren wässrigen oder alkoholischen Lösungen eingesetzt. Die eingesetzten Mengen an alkalischem Katalysator betragen im allgemeinen 0,2 bis 20,0 Mol-%, bezogen auf Silikonorganopolymer.

Die Verseifung wird im Allgemeinen bei Temperaturen von 20°C bis 70°C, vorzugsweise 30°C bis 60°C, durchgeführt. Durch Zugabe der Katalysatorlösung wird die Umesterung initiiert. Bei Erreichen des gewünschten Hydrolysegrades, im Allgemeinen zwischen 40 und 100 Mol-%, wird die Umesterung abgebrochen. Bei sauer katalysierter Umesterung erfolgt der Abbruch durch Zugabe von alkalischen Reagenzien. Bei der bevorzugten alkalisch katalysierten Umesterung erfolgt der Abbruch durch Zugabe von sauren Reagenzien, wie Karbonsäuren oder Mineralsäuren. Nach Beendigung der Verseifungsreaktion wird das Produkt von der flüssigen Phase abgetrennt. Dies kann mittels gebräuchlicher Vorrichtungen zur Fest/Flüssig-Trennung erfolgen, beispielsweise mittels Zentrifugation oder Filtration.

Bevorzugt werden Haarpflegezusammensetzungen mit Silikoncopolymeren und/oder deren Verseifungsprodukte bestehend aus:

A) 0,1-50% eines oder mehrerer Silikone, wobei mindestens 50% der Silikone zwei polymerisierbare Gruppen an den Kettenenden aufweisen.
B) 0,5-14% eines oder mehrerer hydrophiler Monomere
C) 30-99,4% eines Vinylesters oder eines Esters der Acryl- oder Methacrylsäure als hydrophobes Monomer und gegebenenfalls weiteren hydrophoben Monomeren,

wobei dass Molokolargewicht $M_W$ das Silikonpolymers 30.000 bis 90.000 g/mol beträgt.

Besonders bevorzugt werden Haarpflegezusammensetzungen mit Silikoncopolymeren und/oder deren Verseifungsprodukte bestehend aus:

A) 0,1-40% eines oder mehrerer Silikonmakromonomere mit der allgemeinen Formel (1):

$$R^1R_2SiO(SiR_2O)_nSiR_2R^1 \qquad\qquad (1)$$

wobei

R unabhängig von einander, einen einwertigen, linearen oder zyklischen, Si-C-gebundenen, gegebenenfalls substituierten Kohlenwasserstoffrest oder einen Alkoxyrest mit 1 bis 18 Kohlenstoffatom(en) je Rest

$R^1$ eine polymerisierbare Gruppe bedeutet

n 10 bis 1000

bedeuten

B) 0,5-10% einer radikalisch polymerisierbaren Carbonsäure ausgewählt aus der Gruppe enthaltend Crotonsäure oder Acrylsäure und gegebenenfalls weiteren hydrophilen Monomeren

C) 30-99,4% Vinylacetat als hydrophobes Monomer und gegebenenfalls weiteren hydrophoben Monomeren, mit der Massgabe, dass das Molokulargewicht $M_w$ des Silikonpolymers 30.000 bis 90.000 g/mol beträgt.

Die erfindungsgemäßen Silikoncopolymere können beispielsweise in einer radikalischen Lösungspolymerisation in einem Lösungsmittel oder Lösungsmittelgemisch synthetisiert werden, dass über gute Löseigenschaften sowohl für den Silikon- als auch für den organischen Anteil verfügt und gleichzeitig als Molekulargewichtsregler wirkt.

Die nach diesem bevorzugten Verfahren erhaltenen Silikoncopolymere zeichnen sich durch hohe Transparenz und fehlende Phaseseparation aus. TEM-mikroskopisch sind entweder keine diskreten Silikondomänen oder lediglich sehr kleine Silikondomänen von weniger als 300 nm in der kontinuierlichen Matrix zu erkennen. Überraschenderweise wurde gefunden, dass die Silikoncopolymere gegenüber den beispielsweise aus der EP 0 412 704 B1 bekannten, phasen-separierten Silikon-Propfcopolymeren, nochmals deutlich verbesserte Eigenschaften aus der Kombination von Halt und Weichgriff aufweisen.

Die erfindungsgemäße Haarpflegezusammensetzung zeigt Ihre Vorteile besonders bei Haarstyling Anwendungen, da sie die bisherige Einschränkung überwindet und durch ihre Zusammensetzung auch Formulierungen zugänglich macht, die den scheinbar gegensätzlichen Zusammenhang zwischen großen Tröpfchen während des Sprühvorganges und niedrige Viskosität auf der Haarfaser ermöglicht. Wobei die Haarspray Anwendungen guten Halt und guten Haarweichgriff(-gefühl) durch die Verwendung mindestens eines einzelnen Silikoncopolymers ermöglichen. Ein weiterer Vorteil ist die Möglichkeit einer wässrigen Formulierung bei einem Anteil von weniger als 15% hydrophilem Monomer B). Diese führt ebenfalls zu einer Erhöhung des Weichgriffs und der Flexibilität.

Außerdem bieten die erfindungsgemäßen Silikoncopolymere den Vorteil insbesondere auch umweltfreundliche Aerosol- und Pumpspray Formulierungen mit einem Wassergehalt von mindestens 10% aufzubauen, ohne einen zusätzlichen Kohlenwasserstoff als Lösungsmittel mit einem Siedpunkt von mehr als 105°C zu benötigen.

Die Haarpflegezusammensetzung beinhaltet 0,1-12 Gew.-% mindestens eines der erfindungsgemäßen Silikoncopolymere und/oder dessen Verseifungsprodukte. Besonders bevorzugt ist der Bereich 2-8 Gew.-% und ganz besonders bevorzugt 2,5-6 Gew.-%.

Im erfindungsgemäßen Silikoncopolymer werden durch die Verwendung von di- und/oder multifunktionellen Silikonmakromonomeren A) die organischen Polymerketten durch Silikonketten kovalent verbrückt. Erfindungswesentlich ist, dass die Bildung unlöslicher Netzwerke verhindert wird. Dies gelingt auf dem Fachmann bekannte Weise, beispielsweise durch Kontrolle des Molekulargewichts und/oder Anpassung des Festgehalts während der Polymerisation. Die erfindungsgemäßen Silikoncopolymere haben ein Molekulargewicht Mw von mindestens 30000 g/mol *und höchstens 90000g/mol. Besonders bevorzugt liegt das Molekulargewicht Mw zwischen 35000-60000 g/mol. Die organischen Blöcke sind dabei aus unterschiedlichen Monomeren aufgebaut. Sie enthalten sowohl hydrophile Monomere B) als auch hydrophobe Monomere C).

Geeignete Silikone A) sind lineare oder verzweigte Polysiloxane mit einer Kettenlänge von 10 bis 1000, wobei mindestens 10% der eingesetzten Silikone zwei oder mehr radikalisch polymerisierbare Gruppen aufweisen. Bevorzugt weisen mindestens 50% der Silikone A) zwei polymerisierbare Gruppen an den Kettenenden auf.

Besonders bevorzugt ist mindestens ein Silikon A) welches der allgemeinen Formel (2) entspricht

$$R^1_a R_{3-a}SiO(SiR_2O)_n SiR_{3-a'}R^1_{a'} \qquad (2)$$

wobei

R    gleich oder verschieden ist, und einen einwertigen, gegebenenfalls substituierten, linearen oder verzweigten Alkylrest oder Alkoxyrest mit jeweils 1 bis 18 C-Atomen bedeutet, wie beispielsweise Methyl-, Ethyl-, n-Propyl, Isopropyl-, 1-n-Butyl-, 2-n-Butyl, Isobutyl-, tert.-Butyl, n-Pentyl, Isopentyl-, Neopentyl-, tert.-Pentyl, Hexylreste usw., Alkoxyreste wie beispielsweise Methoxy, Ethoxy, Propoxy, n-Butlyoxy usw. Alkyl- und Alkoxyreste R können z.T. auch durch ander Reste substituiert sein wie, Halogen-, Mercapto-, Carboxyl-, Keto-, Enamine-, Amino-, Aminoethylamino-, Aryloxy-, Aryl-, Alkoxysilyl-oder Hydroxylreste.

$R^1$    eine polymerisierbare Gruppe bedeutet,

a und a'    unabhängig voneinander 0 oder 1 sind,

n    = 10 bis 1000,

bedeutet,

mit der Maßgabe, dass mindestens 10% der eingesetzten Silikone A) zwei polymerisierbare Gruppen besitzen.

Geeignete polymerisierbare Reste $R^1$ sind Alkenylradikale mit 2-8 Kohlenstoffatomen. Beispiele sind polymerisierbare Gruppen wie Vinyl-, Allyl-, Butenyl- und Acrylolyoxyalkyl, Methacryloyloxyalkylgruppe, die Alkylradikale enthält dabei 1-4 Kohlenstoffatome. Bevorzugt sind Vinylgruppen, 3-Methacryloyloxypropyl-, Acryloyloxymethyl- und 3-Acryloyloxypropylgruppsen wie beispielsweise umfassend $\alpha,\omega$-Divinyl-Polydimethylsiloxane, $\alpha,\omega$-Di-(3-acryloxypropyl)-Polydimethylsiloxane, $\alpha,\omega$-Di-(3-methacryloxypropyl)-Polydimethylsiloxane, $\alpha$-Monovinyl-Polydimethylsiloxane, $\alpha$-Mono-(3-acryloxypropyl)-Polydimethylsiloxane, $\alpha$-Mono-(acryloxymethyl)-Polydimethylsiloxane, $\alpha$-Mono-(3-methacryloxypropyl)-Polydimethylsiloxane.

Am meisten bevorzugte Silikone A sind sortenreine $\alpha,\omega$-Divinylpolydimethylsiloxane der allgemeinen Formel $CH_2=CH-SiMe_2(SiMe_2O)_nSiMe_2-CH=CH_2$, die beispielsweise von der Wacker Chemie AG, München, Deutschland unter dem Handelsname Polymer PTS-P 1000 vertrieben werden, oder $\alpha,\omega$-Dimethacryloxypropylpolydimethyl-siloxane der allgemeinen Formel $CH_2=C(CH_3)CO-O-(CH_2)_3-SiMe_2(SiMe_2O)_nSiMe_2-(CH_2)_3-O-CO-(CH_3)C=CH_2$, wobei n die obenstehende Bedeutung trägt.

Geeignete hydrophile Monomere B) sind beispielsweise ungesättigte organische Mono- und Polycarbonsäuren, wie zum Beispiel Acrylsäure, Methacrylsäure, Crotonsäure und Dicarbonsäuren wie zum Beispiel Fumarsäure und ungesättigte organische Säureanhydride zum Beispiel Maleinsäureanhydrid, ungesättigte Alkylmethacrylate und deren Mischungen.

Aber auch andere eignen sich als hydrophile Monomere B), wie ungesättigte Carbonsäureamide und Carbonitrile zum Beispiel Acrylamid und Acrylnitril oder auch ungesättigte Sulfonsäuren und deren Salze wie zum Beispiel Vinylsulfonsäure und 2-Acrylamido-2-methylpropansulfonsäure. Kationische Monomere wie Diallyldimethylammoniumchloride (DADMAC), 3-Trimethylammoniumpropyl(meth)acrylamid Chlorid (MAPTAC) und 2-Trimethylammoniumethyl (meth)acrylat chlorid. Besonders bevorzugt werden Crotonsäure, Acrylsäure und Methacrylsäure.

Das Silikoncopolymer und/oder dessen Verseifungsprodukt besteht bevorzugt aus 0,5-10%, besonders bevorzugt aus 3-6%, hydrophilem Monomer B).

Geeignete hydrophobe Monomere C) sind beispielsweise ungesättigte Alkohole und deren Ester wie beispielsweise Vinylester von verzweigten und unverzweigten Alkylcarbonsäuren mit 1-15 Kohlenstoffatomen. Besonders bevorzugt sind Vinylacetat, Vinylpropionat, Vinylbutyrat, Vinyl 2-ethylhexanoat, Vinyllaurat, 1-Methylvinylacetat, Vinylpivalat und Vinylester von alpha-verzweigten Monocarbonsäuren mit 5-11 Kohlenstoffatomen wie zum Beispiel VeoVa9 und VeoVa10 (Handelsnamen von Hexion Specialty Chemicals, Columbus, Ohio, USA). Am meisten bevorzugt wird Vinylacetat.

Weitere hydrophobe Monomere C) können ungesättigte Kohlenwasserstoffe wie beispielsweise Ethen, Propen oder Buten und Isobutene sein.

Zudem sind als hydrophobe Monomere C) auch die folgenden Stoffe geeignet. Acrylsäure- und Methacrylsäureester und deren Mischungen wie beispielsweise Ester von verzweigten und unverzweigten Alkoholen mit Kohlenwassertsoffatomen von 1-15. Dabei werden besonders bevorzugt Methylmethacrylat, Methylacrylat, Ethylacrylat, Ethylmethacrylat, Propylacrylat, Propylmethacrylat, n-,iso,und t-Butylacrylat, n-, iso- and t-Butylmethacrylat, 2-Ethylhexylacrylat und Norbonylacrylat. Ebenso die Diester von Fumar- und Maleinsäure wie zum Beispiel Diethyl- und Disopropylester.

Speziell für Aerosol Haarsprays mit umweltfreundlicher Zusammensetzung, beispielsweise für den Gebrauch in den USA, müssen Systeme mit einem niedrigen Gehalt an organischen flüchtigen Inhaltsstoffen (low volatile organic compound - kurz- VOC) entwickelt werden aufgrund des sogenannten Clean Air Act. Dieser Umstand macht wässrige Systeme besonders interessant.

Außerdem sollte für eine einfache Anwendung auf/in nassem Haar das Lösungsmittel mit Wasser mischbar sein und das Stylingpolymer sollte keine Phasentrennung zeigen oder ausgefällt werden, wenn Wasser in das System eingetragen wird.

Es bietet sich also an, ein wässriges Lösungsmittel auszuwählen, um eine einfache Anwendbarkeit in nassem Haar zu ermöglichen und den Anteil an flüchtigen, organischen Komponenten zu limitieren.

Die Haarsprayzusammensetzung enthält ein passendes Lösungsmittel oder -gemisch welches mehr als 3% Wasser enthält und in der Sprayzusammensetzung zwischen 25-98% vorliegt.

Wässrig alkoholische Lösungsmittelgemische enthalten mindestens 2,94-88% Wasser und 12-97,06% eines Alkohols oder Mischungen aus Alkoholen und alkohollöslichen kosmetisch akzeptablen Lösungsmitteln wie Silikone, Alkane und verzweigte Alkane, Alkylcarbonate, Alkyllactate, Aceton, Dialkylether oder Alkylester. Beispielhaft aber nicht limitierend seien aufgeführt: Ethanol, Isopropanol, Pentane, lineare flüchtige Silikone wie beispielsweise Wacker-Belsil DM 1 plus, SLM 28032, SLM 28033 und SLM 28038 oder zyklische Silikone wie Wacker-Belsil CM040 (Wacker Chemie AG, München, Deutschland), Dicaprylylcarbonat oder Butyllactat.

Bevorzugt werden Lösungsmischungen aus 15-60% Wasser und 40-85% Ethanol.

Die eingesetzten Silikoncopolymere, bei denen als hydrophile Monomere, Vertreter mit Säurefunktionen eingesetzt wurden, müssen in den Haarsprayanwendungen neutralisiert oder teilneutralisiert werden von 40-100%. Bevorzugt ist der Bereich von 70-100%. Als Basen zur Neutralisation werden organische Basen wie Aminoalkohole wie beispielsweise 2-Amino-2-Methyl-1-propanol eingesetzt. Neben den organischen Basen können auch anorganische Basen wie beispielsweise Kaliumhydroxid eingesetzt werden. Auch Mischungen aus anorganischen und organischen Basen können eingesetzt werden. Die Menge an Base, die zum gewünschten Neutralisierungsgrad des Polymers benötigt wird, lässt sich mit der Gleichung (I) berechnen,

$$B[g] = \frac{S[\frac{mg}{g}] * b[\frac{g}{mol}] * \frac{N}{100} * P[g]}{56{,}11[\frac{g}{mol}] * \frac{c}{100} * 1000} = \frac{S * b * N}{56{,}11 * c * 1000} \qquad (I)$$

wobei

B = Base in g
S = Säurezahl des Polymers [mg KOH/g Polymer]
P = Menge des Polymers [g]
b = Molgewicht der Base [g/mol]
c = Konzentration der Base [%]
N = Neutralisierungsgrad [%]

bedeuten.

In Pumpsprayanwendungen ist kein Treibgas notwendig. Hier wird lediglich das Lösungsmittel benötigt zusammen mit den üblichen Inhaltstoffen wie Neutralisations Agens und weiteren Hilfsstoffen.

Für Aerosole werden in der Haarsprayzusammensetzung 20-50% eines für kosmetische Haaranwendungen passenden Treibgases eingesetzt. Übliche Treibgase aus der Reihe der Kohlenwasserstoffe wie Propan, Butan und Isobutan als auch nicht Kohlenwasserstoffartige Treibgase wie beispielsweise Dimethylether, Kohlendioxid und Stickstoff und deren Mischungen.

Als bevorzugtes Treibgas wird Dimethylether eingesetzt.

Die Haarpflegezusammensetzung kann weitere Zusatzstoffe enthalten, ausgewählt aus der Gruppe enthaltend anionische, kationische oder nichtionische Tenside, Parfums, Lichschutzfilter, Konservierungsmittel, Korrossionschutzmittel, Proteine, Vitamine, Polymere, pflanzliche-, synthethische- oder Mineralöle und jeden beliebigen anderen klassicherweise in kosmetischen Zusammensetzungen verwendeten Zusatzstoff enthaltend beispielsweise Weichmacher zur Variation und Einstellung der Filmeigenschaften wie zum Beispiel Stearate, Citrate, polyetherfunktionelle Silikone, arylfunktionelle Silikone, Glycerin, Fettalkohole, Oleate, Phtalate, Glykole oder Konditioner wie Fettalkohole, andere Silikonfluide und Harze, arylhaltige Silikone zur Glanzverbesserung wie beispielsweise Phenyl trimethicone, Timethylsiloxyphenyl dimethicone mit einem Brechungsindex > 1,46, oder Konditionierende Agentien die der Frisur Volumen verleihen.

Die Herstellung der erfindungsgemäßen Haarpflegezusammensetzung erfolgt nach dem Fachmann bekannten Methoden und ist **dadurch gekennzeichnet, dass** alle Komponenten mit 0,1-12 Gew.-% des erfindungsgemäßen Silikoncopolymers und/oder dessen Verseifungsprodukt vermischt werden, welches aus

A) 0,1-50% eines oder mehrerer Silikone, wobei mindestens 10% dieser Silikone mindestens zwei polymerisierbare Gruppen aufweisen,
B) 0,5-14% eines oder mehrerer hydrophiler Monomere und
C) 30-99,4% eines oder mehrerer hydrophober Monomere besteht.

Die Verwendung der erfindungsgemäßen Haarpflegezusammensetzungen erfolgt als Haarspray, Styling-Mousse, Styling-Gel, Shampoo, Haarspülung, Haarkur, Lotion oder Creme.

**Beispiele**

Herstellung des Silikoncopolymers 1 (erfindungsgemäß) In einem 150 l Rührkessel mit Ankerrührer, Rückflußkühler und Dosiereinrichtungen werden 39,95 kg Ethylacetat, 3,01 kg Isopropanol, 10,19 kg Polymer PTS-P 1000, 1,11 kg Crotonsäure, 0,492 kg VeoVa 10, 3,05 kg Vinylacetat und 0,193 kg Polyphenylvinylen = PPV (tert-Butylperpivalat, 75%ige Lösung in Aliphaten) vorgelegt. Unter Rühren wird auf 70°C aufgeheizt (= Reaktionsbeginn). Zehn Minuten nach Reaktionsbeginn wird die Dosierung einer Mischung aus 1,11 kg Crotonsäure, 3,94 kg VeoVa 10 und 24,41 kg Vinylacetat eingefahren. Die Monomerdosierung erfolgt mit einheitlicher Dosiergeschwindigkeit und erstreckt sich über einen Zeitraum von 4 h. Dreißig Minuten nach Reaktionsbeginn werden 0,075 kg PPV zugeben. Weitere Stoßdosierungen des Initiators erfolgen im Abstand von 30 min über einen Zeitraum von 5 h (jeweils 0,075 kg PPV, letzte Stoßdosierung 5 h nach Reaktionsbeginn). Nach der letzten Initiatorzugabe wird noch 2 h bei 70°C nachpolymerisiert. Anschließend wird der Ansatz zum Destillieren aufgeheizt. Die erhaltene Polymerschmelze wird mit einer Temperatur von etwa 130°C abgelassen.

Analysen: Säurezahl 30,6 mg KOH/g, Viskosität (Höppler, 10% ige Lösung in Ethylacetat) = 2,0 mPas, SEC $M_w$ = 42414, $M_n$ = 8716, Polydispersität 4,87, $T_g$ = 39,2°C.

Herstellung des Silikoncopolymers 2 (erfindungsgemäß) In einem 3 l Laborreaktor werden 649,77 g Ethylacetat, 48,91 g Isopropanol, 251,09 g Polymer PTS-P 1000, 27,29 g Crotonsäure, 12,12 g VeoVa 10, 75,13 g Vinylacetat und 4,77 g PPV vorgelegt. Unter Rühren wird auf 70°C aufgeheizt (= Reaktionsbeginn). Zehn Minuten nach Reaktionsbeginn wird die Dosierung einer Mischung aus 27,29 g Crotonsäure, 97,05 g VeoVa 10 und 601,71 g Vinylacetat eingefahren. Die Monomerdosierung erfolgt mit einheitlicher Dosiergeschwindigkeit und erstreckt sich über einen Zeitraum von 4 h. Dreißig Minuten nach Reaktionsbeginn werden 1,85 g PPV zugeben. Weitere Stoßdosierungen des Initiators erfolgen im Abstand von 30 min über einen Zeitraum von 5 h (jeweils 1,85 g PPV, letzte Stoßdosierung 5 h nach Reaktionsbeginn). Nach der letzten Initiatorzugabe wird noch 2 h bei 70 °C nachpolymerisiert. Anschließend wird der Ansatz zum Destillieren aufgeheizt.

Analysen: Säurezahl 32,4 mg KOH/g, Viskosität (Höppler, 10% ige Lösung in Ethylacetat) = 2,4 mPas, SEC $M_w$ = 86605, $M_n$ = 11567 Polydispersität 7,49, $T_g$ = 37,9°C.

Herstellung des Silikonpolymers 3 (nicht erfindungsgemäß) In einem 150 l Rührkessel mit Ankerrührer, Rückflußkühler und Dosiereinrichtungen werden 31,59 kg Ethylacetat, 5,88 kg Isopropanol, 0,408 kg Dehesive 929, 1,47 kg Vinylacetat und 0,022 kg PPV vorgelegt. Unter Rühren wird auf 70°C aufgeheizt (= Reaktionsbeginn). Nach Erreichen der Innentemperatur von 70°C wird die Initiatordosierung (0,107 kg PPV in 2,76 kg Ethylacetat) über 310 Minuten gestartet. Zehn Minuten nach Reaktionsbeginn wird die Monomerdosierung (3,27 kg Dehesive 929, 11,76 kg Vinylacetat) über 4h gestartet. Nach Ende der beiden Dosierungen wird noch zwei Stunden bei 70°C auspolymerisiert. Anschließend wird der Ansatz zum Destillieren aufgeheizt. Die erhaltene Polymerschmelze wird mit einer Temperatur von ca. 130°C abgelassen.

Analysen: Säurezahl 0,561 mg KOH/g, Viskosität (Höppler, 10% ige Lösung in Ethylacetat) = 1,33 mPas, SEC $M_w$ = 14255, $M_n$ = 4912, Polydispersität 2,90, $T_g$ = 27,0°C.

Herstellung des Silikoncopolymers 4 (erfindungsgemäß) In einem 150 l Rührkessel mit Ankerrührer, Rückflußkühler und Dosiereinrichtungen werden 26,47 kg Ethylacetat, 5,57 kg Isopropanol, 4,43 kg VIPO 300, 2,33 kg Crotonsäure, 2,61 kg VeoVa 10, 11,19 kg Vinylacetat und 0,163 kg PPV vorgelegt. Unter Rühren wird auf 70°C aufgeheizt (= Reaktionsbeginn). Bei Reaktionsbeginn wird mit der Dosierung von 0,63 kg PPV in 5,59 kg Ethylacetat über einen Zeitraum von 510 min begonnen. Zehn Minuten nach Reaktionsbeginn wird die Dosierung einer Mischung aus 1,40 kg Crotonsäure, 1,12 kg VeoVa 10, 5,59 kg Vinylacetat und 2,98 kg VIPO 300 (ein $\alpha,\omega$-Divinylsilikon mit mittlerer Kettenlänge 133) eingefahren. Die Monomerdosierung erfolgt mit einheitlicher Dosiergeschwindigkeit und erstreckt sich über einen Zeitraum von 120 Minuten. 20 Minuten nach Ende dieser Monomerdosierung wird eine weitere Monomerdosierung (5,59 kg Vinylacetat, 1,17 kg VIPO 300) über 300 Minuten gestartet. Nach Ende aller Dosierungen wird noch 2 h bei 70°C nachpolymerisiert. Anschließend wird der Ansatz zum Destillieren aufgeheizt.

Analysen: Säurezahl 67,1 mg KOH/g, Viskosität (Höppler, 10% ige Lösung in Ethylacetat) = 1,5 mPas, SEC $M_w$ = 26049, $M_n$ = 7670 Polydispersität 3,4, $T_g$ = 46,0°C.

Die oben beschriebenen Silikoncopolymere 1, 2, 3 und 4 werden im Folgenden in Haar-Styling Formulierungen / Zusammensetzungen eingesetzt. Diese folgenden Beispiele sind Grundrezepturen, welche mit weiteren Zusatzstoffen, wie oben aufgeführt, nach dem Fachmann bekannten üblichen Konzentrationen erweitert werden können.

Herstellung Aerosol Haar-Spray: Silikoncopolymere werden im Lösungmittel oder Lösungsmittelgemisch wie beispielsweise Wasser/Ethanol unter Rühren gelöst. Bei Silikoncopolymeren die Säuregruppen tragen, wird dem Lösungsmittel oder Lösungsmittelgemisch zuerst die entsprechende Menge an Base, wie beispielsweise Aminomethylpropanol- die je nach Neutralisierungsgrad berechnet wurde- zugesetzt. Das Silikoncopolymer wird dann unter. Rühren gelöst. Bei Zusatz weiterer Additive werden diese ebenfalls unter Rühren gelöst. Je nach Additiv wird die Lösung durch Erwärmen bis zu 40°C schneller erhalten.

Die erhaltene Polymerlösung wird in die Aerosoldosen abgefüllt und in einer Aerosolabfüllanlage mit dem entsprechenden Treibgas oder Treibgasgemisch bestückt.

Die Herstellung von Pump-Haarspray erfolgt analog. Jedoch benötigt man kein Treibgas. Die Polymerlösungen werden in Pumpsprayverpackungen abgefüllt.

Beispiel 1: Aerosol-Haar-Spray mit 3 Gew.-% Silikoncopolymer

Die Zusammensetzung des Haar-Sprays ist in der Tabelle 1 wiedergegeben.

Tabelle 1

| Stoff | Konzentration [Gew.-%] |
|---|---|
| Ethanol | 34,43 |
| Aminomethylpropanol 30%ig | 0,46 |
| Wasser | 22,11 |
| Silikoncopolymer 1 oder 2 oder 3 | 3,00 |
| Dimethylether | 40,00 |

Beispiel 2: Aerosol-Haar-Spray mit 4,5 Gew.-% Silikoncopolymer

Die Zusammensetzung dieser Haar-Sprays ist in der Tabelle 2 wiedergegeben.

Tabelle 2

| Stoff | Konzentration [Gew.-%] |
|---|---|
| Ethanol | 33,33 |
| Aminomethylpropanol 30%ig | 0,67 |
| Wasser | 21,50 |
| Silikoncopolymer 1 oder 2 oder 3 | 4,50 |
| Dimethylether | 40 |

Beispiel 3: Aerosol-Haar-Spray mit 6 Gew.-% Silikoncopolymer Die Zusammensetzung dieser Haar-Sprays ist in der Tabelle 3 wiedergegeben.

Tabelle 3

| Stoff | Konzentration [Gew.-%] |
|---|---|
| Ethanol | 37,58 |
| Aminomethylpropanol 30%ig | 0,35 |
| Wasser | 24,07 |
| Silikoncopolymer 1 oder 2 oder 3 | 6,00 |
| Dimethylether | 32,00 |

Beispiel 4: Pump-Haar-Sprays mit 10 Gew.-% Silikoncopolymer

Die Zusammensetzung dieser Pump-Haar-Sprays ist in der Tabelle 4 wiedergegeben.

Tabelle 4

| Stoff | Konzentration [Gew.-%] |
|---|---|
| Ethanol | 54,30 |
| Aminomethylpropanol 30%ig | 0,80 |
| Wasser | 34,90 |

(fortgesetzt)

| Stoff | Konzentration [Gew.-%] |
|---|---|
| Silikoncopolymer 1 oder 2 oder 3 | 10,00 |

Herstellung von Styling Mousse:

In Wasser wird die Base, wie beispielsweise Aminomethylpropanol, zusammen mit dem Emulgator, wie beispielsweise PEG-40 Hydrogenated Castor Oil, unter Rühren gelöst. Diese Mischung wird auf max. 50°C erwärmt und das Silikoncopolymer portionsweise unter Rühren darin gelöst. Je nach Empfindlichkeit gegenüber Wärme - werden weitere Additive bei 50°C oder nach Abkühlen bei Raumtemperatur zugegeben und unter weiterem Rühren gelöst. Die abgekühlte Lösung wird in Dosen abgefüllt und mit dem Treibgas oder Treibgasgemisch bestückt.

Beispiel 5: Styling Mousse mit 3 Gew.-% Silikoncopolymer.

Die Zusammensetzung dieser Styling Mousse ist in der Tabelle 5 wiedergegeben.

Tabelle 5

| Stoff | Konzentration [Gew.-%] |
|---|---|
| Aminomethylpropanol 30%ig | 1,06 |
| Wasser | 74,80 |
| PEG-40 Hydrogenated Castor Oil (Cremophor RH 40 von BASF AG , Ludwighafen, Deutschland) | 0,50 |
| Silikoncopolymer 4 | 3,00 |
| Polyquaternium-10 (UCARE Polymer JR 400 der Firma Amerchol Corporation, Piscataway, USA) | 0,80 |
| Amodimethicone, Cetrimonium Chloride, Trideceth-10 (Wacker-Belsil® ADM 6057 E, der Firma Wacker Chemie AG, München, Deutschland) | 0,30 |
| Cocamidopropyl Betaine (Genagen CAB 818 30% der Firma Clariant GmbH, Frankfurt, Deutschland) | 11,04 |
| Phenoxyethanol, Methylparaben, Ethylparaben, Butylparaben, Propylparaben und Isobutylparaben (Phenonip der Firma Schülke & Mayr GmbH,Norderstedt, Deutshcland) | 0,50 |
| Propan/Butan | 8,00 |

Die so erhaltenen Haarpflegezusammensetzungen wurden verschiedenen Tests unterzogen die Ihre Eigenschaften auf Haaren zeigen.

**Curl retention**

And diesem Modell zur Ermittlung der Haarfixierung bei hohen Luftfeuchtigkeiten werden die prozentualen Veränderungen von Ausgangs- zu Endlänge von definiert präparierten Locken im Vergleich zu Haartressenlänge aufgezeichnet. Die Curl Retention Eigenschaften von Haar Styling Produkten werden bei 23°C und 90% relative Luftfeuchtigkeit über einen Zeitraum von 24 Stunden verfolgt.

Es werden aus braunem europäischem Haar 15 cm Länge Haartressen als Bündel a 3,5 g zusammengestellt und mit einem Bindfaden gebunden und mit einem geeignetem Klebstoff dauerhaft fixiert. Die Haartressen werden mit Shampoo gewaschen und mit vollentsaltztem (=VE) Wasser ausgewaschen. Die Haartressen werden gekämmt und auf einen Kunststoffstab mit einem Durchmesser von 1,4 cm gewickelt, mit einer Hülle temporär fixiert und bei 50°C über Nacht getrocknet. Die Locken werden von den Kunststoffstäben vorsichtig abgestreift und anschließend nach dem Abkühlen mit dem zu testenden Spray gleichmäßig besprüht. Nach einer einstündigen Trocknungsphase bei Raumtemperatur werden die Locken an einer skalierten Hängevorrichtung in einen Klimaschrank bei 23°C und 90% relativer Luftfeuchtigkeit befestigt. Zuvor wurde die Ausganglänge der Locke bestimmt und notiert. In bestimmten Zeitintervallen werden die Lockenlängen, das heißt die Veränderung zur Ausgangslänge über einen Zeitraum von 24 h abgelesen.

Die Berechnung erfolgt nach Gleichung (II),

$$\% \ CURL \ RETENTION = \frac{L - Lt}{L - L0} \ x \ 100 \qquad\qquad (II)$$

wobei m

L = Länge der Haartresse
$L_o$ = Ausgangslänge der Locke
$L_t$ = Länge der Locke nach/während der Messung
bedeuten.

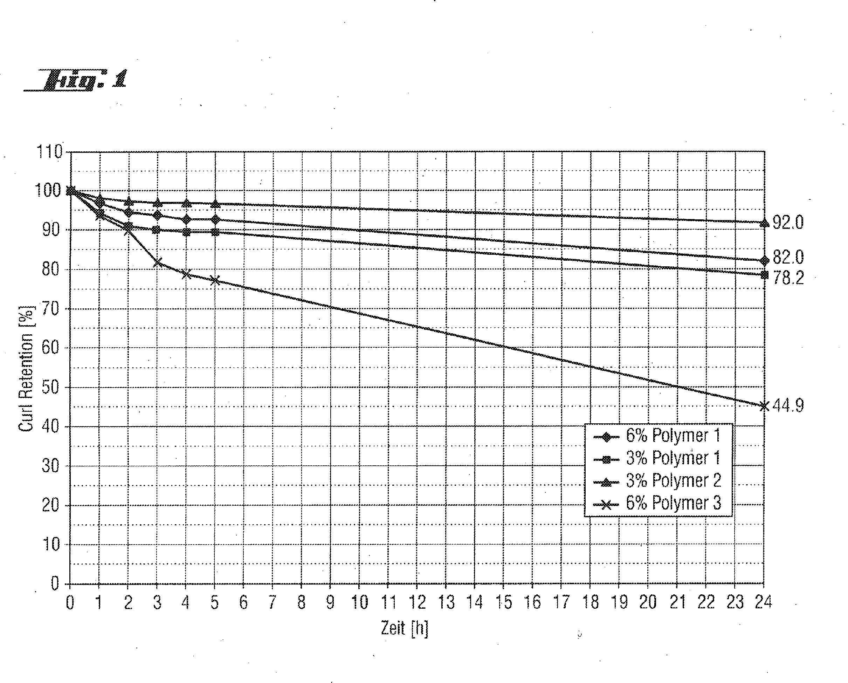

Die Figur 1 zeigt beispielhaft die Ergebnisse dieses Tests für die erfindungsgemäßen Beispiele 1 mit 3 Gew.-% Silikoncopolymer 1 und 2, sowie Beispiel 3 mit 6 Gew.-% Silikoncopolymers 1 im Vergleich zu dem nicht erfindungsgemäßen Beispiel 3 mit 6 Gew.-% Silikoncopolymer 3.

Bei weiteren analog durchgeführten Versuchen ergab sich, dass erfindungsgemäße Silikoncopolymere bei Einsatzkonzentration von 2-12 Gew.-% gute Curl retention Werte zeigen. Insbesondere ab 3 Gew.-% erhält man einen sehr guten Halt bei hoher Luftfeuchtigkeit, der selbst nach 24 h noch Curl retention Resultate im Bereich 75-95% liefert. Das nicht erfindungsgemäße Beispiel 3 mit 6 Gew.-% Silikoncopolymers 3 zeigt dahingegen einen sehr starken Abfall auf etwa 45% innerhalb dieser Zeit.

### Elastizität/Flexibilität/Plastizität

Zur Bestimmung und Beurteilung der Flexibilität und Elastizität des Polymerfilms im Haar wird die Drei-Punkt-Biegesteifigkeit bestimmt. Haartressen aus braunem, europäischem Haar von 20 cm Länge werden gewaschen und getrocknet. Die Haare werden in einem Abstand von 20 cm Entfernung gleichmäßig mit Aerosolsprays jeweils 3 sec. auf jeder Seite besprüht. Bei Verwendung eines Pumpsprays erfolgen je 10 Hübe auf jeder Seite. Pro Produkt werden 5 Tressen verwendet. Die behandelten Tressen werden 24 h im Klimaraum konditioniert.

Die Untersuchungen der erfindungsgemäßen Haarpflegezusammensetzungen zeigen einen sehr flexiblen Polymerfilm der sehr gutes elastisches Verhalten aufweist. Die Ergebnisse können Idealwerte von 1,00 liefern oder sind sehr nahe am Idealwert für elastisches und flexibles Verhalten wie in der Literatur beschrieben. Details der Messapparatur und der Messmethodik sind dem Fachmann bekannt und werden beispielsweise in den folgenden Literaturstellen beschrieben, deren diesbezügliche Offenbarung auch Gegenstand dieser Anmeldung sein soll: Dynamic hairspray analysis. I. Instrumentation and preliminary results, J. Jachowicz , Y. Kao, J. Soc.Cosmet. Chem , p.73, 47 (1996); Dynamic hair spray analysis. II. Effect of polymer, hair type, and solvent composition, J. Jachowicz ; Y. Kao, J. Soc.Cosmet. Chem , p.281, 52 (2001); Mechanical alalysis of elasticity and flexibility of virgin and polymer-treated hair fiber assemblies, J. Jachowicz, R. McMullen, J. Cosmet. Sci., p.345, 53 (2002).

### Klebrigkeit und Trocknungszeit

Während der Anwendung eines Haarsprays entweicht beziehungsweise verflüchtigt sich ein Teil des Lösungsmittels. In dieser Zeit verändert sich auch das Empfinden bei Berührung der Haare von klebrig und nicht-klebrig. Der Effekt beruht auf der Weichmacherfunktion des Lösungsmittels und hängt von verschiedenen Faktoren ab wie beispielsweise Gewicht des Sprays, Temperatur und Luftzirkulation. Bei diesem einfachen Test werden die genannten Faktoren konstant gehalten um einen Vergleich zwischen verschiedenen Formulierungen zu ermöglichen. Man verwendet 15 cm lange, 3,5g schwere Haartressen aus braunem, europäischem Haar. Die Haartresse wird in 10 cm Abstand 4 Sekunden besprüht und dabei gedreht. Direkt nach dem Sprühvorgang, werden die Haartressen wiederholt von oben nach unten betastet, bis die Haartresse vollständig als trocken empfunden wird. Dabei werden folgende Zeiten registriert:

a) Zeit vom Start bis die Haartresse als klebrig empfunden wird
b) Zeit vom Start bis die Haartresse als nicht mehr klebrig empfunden wird
c) Zeit vom Start bis die Haartresse als völlig getrocknet bewertet wird.

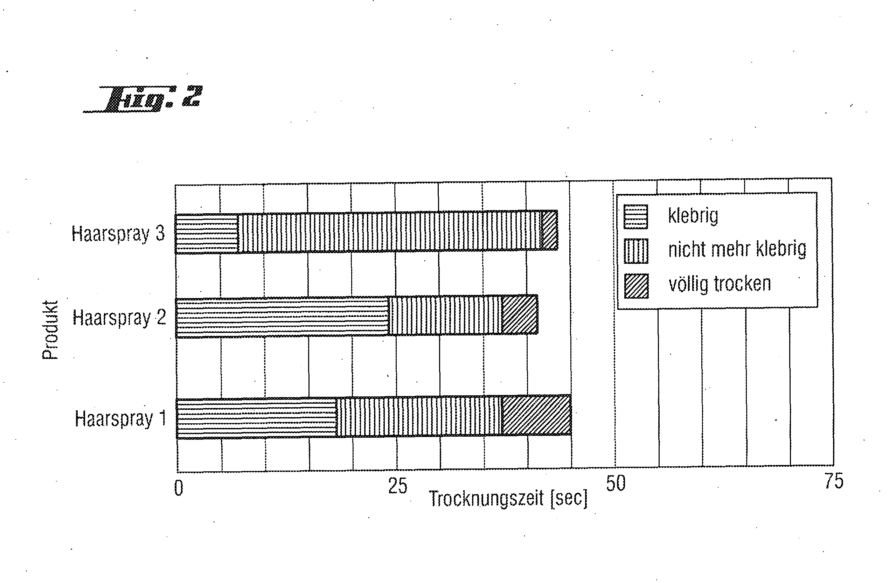

Die Tests werden mehrmals wiederholt. Figur 2 zeigt die Ergebnisse für 5 Beispiele. Haarspray 1 entspricht der Beispielsrezeptur 3 bei dem das Silikoncopolymer 3 eingesetzt wurde. Haarspray 2 entspricht der Beispielsrezeptur 1 bei der das Silikoncopolymer 1 eingesetzt wurde und Haarspray 3 entspricht der Beispielsrezeptur 3 bei der Silikoncopolymer 1 eingesetzt wurde.

Im Paneltest werden schnelle Trocknungszeiten der erfindungsgemäßen Beispiele festgestellt. Die Zeitphase in der das besprühte Haar als klebrig empfunden wird ist trotz eines höheren Wassergehaltes in den Rezepturen, mit einer Dauer zwischen 20-30 Sekunden, sehr kurz.

**Paneltest**

In einem Paneltest bekommen 12 Testpersonen jeweils 6 behandelte Haartressen a 20cm und 3,5g von braunem, europäischem Haar. Die Haartressen werden in einem Abstand von 15 cm auf jeder Seite je zwei Sekunden (Aerosol) oder mit 4 Hüben (Pumpspray) besprüht. Die Tressen werden über Nacht im Klimaraum bei 23°C und 60% relativer Luftfeuchte konditioniert. Eine Haartresse ist eine bekannte Referenz. Die Haartressen sind mit einer dreistelligen Codierung gekennzeichnet, so dass die Probanden keine Kenntnis des Produktes bekommen. Jeder Proband bekommt ein individuelles Tressenset. Die Proben werden von den Probanden in eine Reihenfolge gebracht und mit einer Bewertung versehen. Es werden dabei folgende Eigenschaften bewertet:

Steifheit / Flexibilität

Bewertet wird mit einer fünfstelligen Skala beispielsweise für die Flexibilität sehr steif (1) bis sehr flexibel (5) oder für die Steifheit dann für sehr flexibel (1) und für sehr steif (5).

Angenehmer Haarweichgriff vor dem Kämmen

Bewertet wird auf einer Skala von 1-5 wie angenehm der Haargriff empfunden wird. Von sehr hart/unnatürlich (1) bis sehr weich/natürlich (5).

Knistern (und Brechen des Polymerfilms)

Bewertet wird auf einer Skala von 1-5 wie laut der Polymerfilm oder die Fixierungspunkte bricht/brechen. Von sehr laut (1) bis sehr leise (5).

Adhäsion: Halt und Vernetzung

Die Probanden ziehen die Haartressen horizontal auseinander und bewerten wie gut eine Vernetzung der Haarfasern durch das Haarspray aufgebaut wird und wie gut diese haftet auf einer Skala von 1-5: keine Vernetzung/ kurzfristiger Halt (1) bis sehr gute Vernetzung (dauerhafter Halt = 5).

Trockenkämmbarkeit

Nach Beurteilung der Punkte Knistern und Adhäsion werden die Haartressen mit einem schwarzen Kamm durchgekämmt. Die Leichtigkeit des Durchkämmens wird dabei auf einer Skala von 1-5 bewertet.

| | |
|---|---|
| 5 | keinen oder nur sehr geringen Widerstand beim Durchkämmen |
| 4 | mäßigen Widerstand beim Durchkämmen |
| 3 | mäßigen bis deutlichen Widerstand beim Durchkämmen |
| 2 | starken Widerstand beim Durchkämmen |
| 1 | nicht kämmbar |

Schuppenbildung

Während des Durchkämmens kann das Polymer von den Haaren abgestreift werden und als Rückstand anfallen, der ähnlich einer Schuppenbildung aussieht. Die Beurteilung findet zeitgleich mit der Trockenkämmbarkeit statt. Die Probanden beurteilen visuell das Auftreten von Rückständen auf dem schwarzen Kamm auf einer Skala von 1-5. Von einem deutlichem sichtbaren Rückstand (1) bis nicht sichtbaren Rückstand (5).

Angenehmer Haargriff nach dem Kämmen

Bewertet wird auf einer Skala von 1-5 wie angenehm der Haargriff empfunden wird. Von sehr hart/unnatürlich (1) bis sehr weich/natürlich (5).

Statische Aufladung

Die Haartressen werden 2 h im Klimaraum bei 23°C und 60% Luftfeuchte konditioniert. Danach werden die Tressen dreimal aufeinander folgend rasch und kräftig durchgekämmt. Das Ausmaß der statischen Aufladung (Fly-Away effect) wird visuell auf einer Skala von 1-5 bewertet. Von starkem Fly-Away Effekt (1) bis kein Fly-Away Effekt (5).

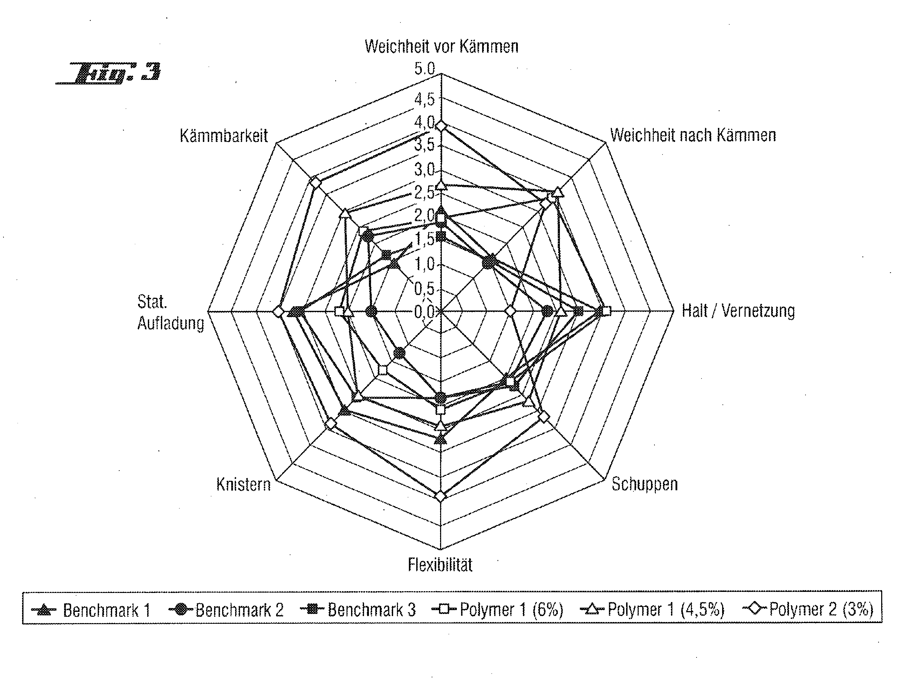

Zusammenfassend ergab sich aus diesen Tests die Figur 3. Sie zeigt das Ergebnis des Vergleichs der erfindungsgemäßen Zusammensetzungen mit Silikoncopolymeren 1 und 2 in Konzentrationen von 3 Gew.-%, 4,5 Gew.-% und 6 Gew.-% mit kommerziellen Produkten (Benchmark 1 bis 3). Dabei zeigten die erfindungsgemäßen Zusammensetzungen eine deutliche Verbesserung der Kombination aus Halt, Flexibilität und weichem angenehmen Griff, der einen natürlichen Halt ausmacht. Während die kommerziellen Produkte Silikone als Konditioner in der Rezeptur einsetzen, um das gezeigte Verhalten erlangen zu können, können die erfindungsgemäßen Silikoncopolymere wegen ihres Hybridcharakters, durch Variation der Einsatzmenge, auf eine gewünschtes Profil an Halt und Griffweichheit eingestellt werden.

## Patentansprüche

1. Haarpflegezusammensetzungen, **dadurch gekennzeichnet, dass** sie 0,1-12 Gew.-% mindestens eines Silikoncopolymers oder/und dessen Verseifungsprodukte enthalten, welches aus

   A) 0,1-50% eines oder mehrerer Silikone, wobei mindestens 10% dieser Silikone mindestens zwei polymerisierbare Gruppen aufweisen,
   B) 0,5-14% eines oder mehrerer hydrophiler Monomere und
   C) 30-99,4% eines oder mehrerer hydrophober Monomere besteht,
   mit der Massgabe, dass das Molekulargewicht $M_w$ des Silikoncopolymers 30.000 bis 90.000 g/mol beträgt.

2. Haarpflegezusammensetzungen gemäß Anspruch 1, **dadurch gekennzeichnet, dass** mindestens 50% der Silikone A) zwei polymerisierbare Gruppen an den Kettenenden aufweisen.

3. Haarpflegezusammensetzungen gemäß Anspruch 1 oder 2,
   **dadurch gekennzeichnet, dass** das Silikoncopolymer oder/und dessen Verseifungsprodukte aus 0,1-40% Silikon A) besteht.

4. Haarpflegezusammensetzungen gemäß Anspruch 1 bis 3,
   **dadurch gekennzeichnet, dass** mindestens ein Silikon A) ein Silikonmakromonomer mit der allgemeinen Formel (1) ist

   $$R^1R_2SiO(SiR_2O)_nSiR_2R^1 \qquad (1)$$

   wobei

   R unabhängig von einander, einen einwertigen, linearen oder zyklischen, Si-C-gebundenen, gegebenenfalls substituierten Kohlenwasserstoffrest oder einen Alkoxyrest mit 1 bis 18 Kohlenstoffatom(en) je Rest
   $R^1$ eine polymerisierbare Gruppe bedeutet
   n 10 bis 1000

   bedeuten.

5. Haarpflegezusammensetzungen gemäß Anspruch 1 bis 4,
   **dadurch gekennzeichnet, dass** das Silikoncopolymer oder/und dessen Verseifungsprodukte aus 0,5-10% hydrophiles Monomer B) besteht.

6. Haarpflegezusammensetzungen gemäß Anspruch 1 bis 5,

**dadurch gekennzeichnet, dass** das Silikoncopolymer oder/und dessen Verseifungsprodukte aus 3-6% hydrophilem Monomer B) besteht.

7. Haarpflegezusammensetzungen gemäß Anspruch 1 bis 6,
**dadurch gekennzeichnet, dass** mindestens ein hydrophiles Monomer B) eine radikalisch polymerisierbare Carbonsäure ist, ausgewählt aus der Gruppe enthaltend Crotonsäure oder Acrylsäure.

8. Haarpflegezusammensetzungen gemäß Anspruch 1 bis 4,
**dadurch gekennzeichnet, dass** mindestens ein hydrophobes Monomer C) ausgewählt wird aus der Gruppe enthaltend Vinylester oder Ester der Acryl- oder Methacrylsäure.

9. Haarpflegezusammensetzungen gemäß Anspruch 1 bis 8,
**dadurch gekennzeichnet, dass** mindestens ein hydrophobes Monomer C) Vinylacetat ist.

10. Verfahren zur Herstellung der Haarpflegezusammensetzungen gemäß Anspruch 1 bis 9, **dadurch gekennzeichnet, dass** alle Komponenten mit 0,1-12 Gew.-% mindestens eines Silikoncopolymers und/oder dessen Verseifungsprodukt vermischt werden, welches aus

A) 0,1-50% eines oder mehrerer Silikone, wobei mindestens 10% dieser Silikone mindestens zwei polymerisierbare Gruppen aufweisen,
B) 0,5-14% eines oder mehrerer hydrophiler Monomere und
C) 30-99,4% eines oder mehrerer hydrophober Monomere besteht,
mit der Massgabe, dass das Molekulargewicht $M_w$ des Silikoncopolymers 30.000 bis 90.000 g/mol beträgt.

11. Verwendung der Haarpflegezusammensetzungen gemäß Anspruch 1 bis 9 als Haarspray, Styling-Mousse, Styling-Gel, Shampoo, Haarspülung, Haarkur, Lotion oder Creme.

**Claims**

1. Hair care compositions **characterized in that** they comprise 0.1-12% by weight of at least one silicone copolymer and/or saponification products thereof which consists of

A) 0.1-50% of one or more silicones, where at least 10% of these silicones have at least two polymerizable groups,
B) 0.5-14% of one or more hydrophilic monomers and
C) 30-99.4% of one or more hydrophobic monomers,
with the proviso that the molecular weight $M_w$ of the silicone copolymer is 30,000 to 90,000 g/mol.

2. Hair care compositions according to Claim 1,
**characterized in that** at least 50% of the silicones A) have two polymerizable groups on the chain ends.

3. Hair care compositions according to Claim 1 or 2, **characterized in that** the silicone copolymer and/or saponification products thereof consists of 0.1-40% of silicone A).

4. Hair care compositions according to Claims 1 to 3, **characterized in that** at least one silicone A) is a silicone macromonomer with the general formula (1)

$$R^1R_2SiO(SiR_2O)_nSiR_2R^1 \qquad (1)$$

where

R independently of the others, is a monovalent, linear or cyclic, Si-C-bonded, optionally substituted hydrocarbon radical or an alkoxy radical having 1 to 18 carbon atom(s) per radical
$R^1$ is a polymerizable group
n is 10 to 1000.

5. Hair care compositions according to Claims 1 to 4, **characterized in that** the silicone copolymer and/or saponification products thereof consists of 0.5-10% of hydrophilic monomer B).

6. Hair care compositions according to Claims 1 to 5, **characterized in that** the silicone copolymer and/or saponification products thereof consists of 3-6% of hydrophilic monomer B).

7. Hair care compositions according to Claims 1 to 6, **characterized in that** at least one hydrophilic monomer B) is a free-radically polymerizable carboxylic acid selected from the group comprising crotonic acid or acrylic acid.

8. Hair care compositions according to Claims 1 to 4, **characterized in that** at least one hydrophobic monomer C) is selected from the group comprising vinyl esters or esters of acrylic acid or methacrylic acid.

9. Hair care compositions according to Claims 1 to 8, **characterized in that** at least one hydrophobic monomer C) is vinyl acetate.

10. Method for producing the hair care compositions according to Claims 1 to 9, **characterized in that** all of the components are mixed with 0.1-12% by weight of at least one silicone copolymer and/or saponification product thereof which consists of

A) 0.1-50% of one or more silicones, where at least 10% of these silicones have at least two polymerizable groups,
B) 0.5-14% of one or more hydrophilic monomers and
C) 30-99.4% of one or more hydrophobic monomers,
with the proviso that the molecular weight $M_w$ of the silicone copolymer is 30,000 to 90,000 g/mol.

11. Use of the hair care compositions according to Claims 1 to 9 as hair spray, styling mousse, styling gel, shampoo, hair rinse, hair treatment, lotion or cream.

**Revendications**

1. Compositions de soin capillaire, **caractérisées en ce qu'**elles contiennent 0,1-12 % en poids d'au moins un copolymère de silicone et/ou ses produits de saponification, qui consiste(nt) en

A) 0,1-50 % d'un ou plusieurs silicones, au moins 10 % de ces silicones comportant au moins deux groupes polymérisables,
B) 0,5-14 % d'un ou plusieurs monomères hydrophiles et
C) 30-99,4 % d'un ou plusieurs monomères hydrophobes,
étant entendu que la masse moléculaire $M_w$ du copolymère de silicone va de 30 000 à 90 000 g/mole.

2. Compositions de soin capillaire selon la revendication 1, **caractérisées en ce qu'**au moins 50 % des silicones A) comportent deux groupes polymérisables aux extrémités de la chaîne.

3. Compositions de soin capillaire selon la revendication 1 ou 2, **caractérisées en ce que** le copolymère de silicone et/ou ses produits de saponification consiste(nt) en 0,1-40 % de silicone A).

4. Compositions de soin capillaire selon les revendications 1 à 3, **caractérisées en ce qu'**au moins un silicone A) est un macromonomère silicone de formule générale (1)

$$R^1R_2SiO(SiR_2O)_nSiR_2R^1 \qquad (1)$$

dans laquelle

R représente, indépendamment les uns des autres, un radical hydrocarboné monovalent, linéaire ou cyclique, lié à Si-C, éventuellement substitué, ou un radical alcoxy ayant de 1 à 18 atomes de carbone par radical,
$R^1$ représente un groupe polymérisable
n va de 10 à 1 000.

5. Compositions de soin capillaire selon les revendications 1 à 4, **caractérisées en ce que** le copolymère de silicone et/ou ses produits de saponification consiste(nt) en 0,5-10 % de monomère hydrophile B).

6. Compositions de soin capillaire selon les revendications 1 à 5, **caractérisées en ce que** le copolymère de silicone et/ou ses produits de saponification consiste(nt) en 3-6 % de monomère hydrophile B).

7. Compositions de soin capillaire selon les revendications 1 à 6, **caractérisées en ce qu'**au moins un monomère hydrophile B) est un acide carboxylique polymérisable par voie radicalaire, choisi dans le groupe contenant l'acide crotonique et l'acide acrylique.

8. Compositions de soin capillaire selon les revendications 1 à 4, **caractérisées en ce qu'**au moins un monomère hydrophobe C) est choisi dans le groupe contenant des esters vinyliques et des esters de l'acide acrylique ou méthacrylique.

9. Compositions de soin capillaire selon les revendications 1 à 8, **caractérisées en ce qu'**au moins un monomère hydrophobe C) est l'acétate de vinyle.

10. Procédé pour la préparation des compositions de soin capillaire selon les revendications 1 à 9, **caractérisé en ce qu'**on mélange tous les composants avec 0,1-12 % en poids d'au moins un copolymère de silicone et/ou de son produit de saponification, qui consiste(nt) en

A) 0,1-50 % d'un ou plusieurs silicones, au moins 10 % de ces silicones comportant au moins deux groupes polymérisables,
B) 0,5-14 % d'un ou plusieurs monomères hydrophiles et
C) 30-99,4 % d'un ou plusieurs monomères hydrophobes,
étant entendu que la masse moléculaire $M_w$ du copolymère de silicone va de 30 000 à 90 000 g/mole.

11. Utilisation des compositions de soin capillaire selon les revendications 1 à 9, en tant que laque pour cheveux, mousse de coiffage, gel de coiffage, shampooing, après-shampooing, produit de traitement capillaire, lotion ou crème.

## Fig. 1

EP 1 993 505 B1

Fig. 2

EP 1 993 505 B1

**Fig. 3**

Weichheit vor Kämmen

Kämmbarkeit

Weichheit nach Kämmen

Stat. Aufladung

Halt / Vernetzung

Knistern

Schuppen

Flexibilität

5,0 / 4,5 / 4,0 / 3,5 / 3,0 / 2,5 / 2,0 / 1,5 / 1,0 / 0,5 / 0,0

Benchmark 1 — Benchmark 2 — Benchmark 3 — Polymer 1 (6%) — Polymer 1 (4,5%) — Polymer 2 (3%)

EP 1 993 505 B1

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 0408311 A2
- US 5565193 A
- WO 9500106 A
- WO 9955294 A
- US 6350439 B1
- US 20020015681 A1
- US 5618524 A
- EP 0412704 B1
- WO 0051557 A
- WO 03085035 A1

**In der Beschreibung aufgeführte Nicht-Patentliteratur**

- **R.Y. Lochhead.** *Cosmetic&Toiletries,* 1988, 23, 103
- **J. Jachowicz ; Y. Kao.** *J. Soc.Cosmet. Chem,* 1996, 73, 47
- **J. Jachowicz ; Y. Kao.** *J. Soc.Cosmet. Chem,* 2001, 281, 52
- **J. Jachowicz ; R. McMullen.** *J. Cosmet. Sci.,* 2002, 345, 53